# EUROPEAN PATENT SPECIFICATION

(11) **EP 1 065 997 B1**
(45) Date of publication and mention of the grant of the patent: **14.11.2007**
(21) Application number: 99911406.9
(22) Date of filing: 15.03.1999
(51) Int. Cl.: A61L 27/36

(54) **BONE XENOGRAFTS**
KNOCHENXENOTTRANSPLANTATE
XENOGREFFES OSSEUSES

(30) Priority: 02.04.1998 US 80491 P
(43) Date of publication of application: 10.01.2001
(73) Proprietor: Crosscart Inc., San Francisco, CA 94123 (US); MCP Hahnemann University, Philadelphia, PA 19102-1192 (US)
(72) Inventor: STONE, Kevin, R., Mill Valley, CA 94941 (US); GALILI, Uri, Chicago, IL 60612 (US)
(74) Representative: Kirkham, Nicholas Andrew
(86) International application number: PCT/US1999/005646
(87) International publication number: WO 1999/051170

(56) References cited:
- WO-A-99/44533
- WO-A-99/47080
- US-A- 4 400 833
- US-A- 5 333 626
- US-A- 5 782 915
- GALILI U (REPRINT) ET AL: "High-affinity anti-Gal immunoglobulin G in chronic rejection of xenografts" XENOTRANSPLANTATION, ( AUG 1997 ) VOL. 4, NO. 3, PP. 127-131. PUBLISHER: MUNKSGAARD INT PUBL LTD, 35 NORRE SOGADE, PO BOX 2148, DK-1016 COPENHAGEN, DENMARK. ISSN: 0908-665X., XP009009159 ALLEGHENY UNIV HLTH SCI, DEPT IMMUNOL & MICROBIOL, MED COLL PENN & HAHNEMANN UNIV, 2900 QUEEN LANE, PHILADELPHIA, PA 19129 (Reprint);COLUMBIA PRESBYTERIAN MED CTR, DIV CARDIOTHORAC SURG, NEW YORK, NY 10032; STONE CLIN, SAN FRANCISCO, CA
- GALILI U ET AL: "Porcine and bovine cartilage transplants in cynomolgus monkey: II. Changes in anti-Gal response during chronic rejection." TRANSPLANTATION, (1997 MAR 15) 63 (5) 646-51, XP009009157

## Description

### FIELD OF THE INVENTION

The present invention relates to the field of bone transplantation, and in particular, to replacement and repair of damaged or defective human bone using a substantially immunologically compatible bone from a non-human animal.

### BACKGROUND OF THE INVENTION

Human bone, a hard connective tissue consisting of cells embedded in an extracellular matrix of mineralized ground substance and collagen fibers, (Stedman's Medical Dictionary, Williams & Wilkins, Baltimore, MD (1995)), is the most frequently transplanted tissue in humans. J.M. Lane et al., Current Approaches to Experimental Bone Grafting, 18 Orthopedic Clinics of North America (2) 213 (1987). In the United States alone more than 100,000 bone graft or implant procedures are performed every year to repair or to replace osseous defects resulting from trauma, infection, congenital malformation, or malignancy. *Id.*

Bone grafts and implants are often formed of autologous bone. *Id.* Transplantable autologous bone tissue for large defects, particularly in children, is often unavailable, however. *Id.* In addition, autologous bone transplantation may result in postoperative morbidity such as pain, hemorrhage, wound problems, cosmetic disability, infection or nerve damage at the donor site. *Id.* Further, difficulties in fabricating the desired functional shape from the transplanted autologous bone tissue may result in less than optimal filling of the bone defect *Id.*

Alternatively, much of the structure and many of the properties of original bone tissue may be retained in transplants through use of heterograft or xenograft materials, that is, tissue from a different species than the graft recipient. In the area of soft tissues, for example, tendons or ligaments from cows or other animals are covered with a synthetic mesh and transplanted into a heterologous host in U.S. Pat. No. 4,400,833. Flat tissues such as pig pericardia are also disclosed as being suitable for heterologous transplantation in U.S. Pat. No. 4,400,833. Bovine peritoneum fabricated into a biomaterial suitable for prosthetic heart valves, vascular grafts, bum and other wound dressings is disclosed in U.S. Pat. No. 4,755,593. Bovine, ovine, or porcine blood vessel xenografts are disclosed in WO 84/03036. None of these disclosures describe the use of a xenograft for bone replacement, however.

Once implanted in an individual, a xenograft provokes immunogenic reactions such as chronic and hyperacute rejection of the xenograft, however. In particular, bone xenografts may result in increased rates of fracture, resorption and nonunion secondary to immunologic rejection.

The term "chronic rejection", as used herein, refers to an immunological reaction in an individual against a xenograft being implanted into the individual. Typically, chronic rejection is mediated by the interaction of IgG natural antibodies in the serum of the individual receiving the xenograft and carbohydrate moieties expressed on cells, and/or cellular and/or extracellular matrices of the xenograft. For example, transplantation of cartilage xenografts from nonprimate mammals (*e.g*., porcine or bovine origin) into humans is primarily prevented by the interaction between the IgG natural anti-Gal antibody present in the serum of humans with the carbohydrate structure Galα1-3Galβ1-4G1cNAc-R (α-galactosyl or α-gal epitope) expressed in the xenograft. K.R. Stone et al., Porcine and bovine cartilage transplants in cynomolgus monkey: I. A model for chronic xenograft rejection, 63 Transplantation 640-645 (1997); U. Galili et al., Porcine and bovine cartilage transplants in cynomolgus monkey: II. Changes in anti-Gal response during chronic rejection, 63 Transplantation 646-651 (1997). In chronic rejection, the immune system typically responds within one to two weeks of implantation of the xenograft.

In contrast with "chronic rejection", "hyper acute rejection" as used herein, refers to the immunological reaction in an individual against a xenograft being implanted into the individual, where the rejection is typically mediated by the interaction of IgM natural antibodies in the serum of the individual receiving the xenograft and carbohydrate moieties expressed on cells. This interaction activates the complement system causing lysis of the vascular bed and stoppage of blood flow in the receiving individual within minutes to two to three hours.

The term "extracellular matrix or matrices", as used herein, refer to an extracellular bone matrix of mineralized ground substance and collagen fibers. Stedman's Medical Dictionary, Williams & Wilkins, Baltimore, MD (1995).

Xenograft materials may be chemically treated to reduce immunogenicity prior to implantation into a recipient. For example, glutaraldehyde is used to crosslink or "tan" xenograft tissue in order to reduce its antigenicity, as described in detail in U.S. Pat. No. 4,755,593. Other agents such as aliphatic and aromatic diamine compounds may provide additional crosslinking through the side chain carboxyl groups of aspartic and glutamic acid residues of the collagen polypeptide. Glutaraldehyde and diamine tanning also increases the stability of the xenograft tissue.

Xenograft tissues may also be subjected to various physical treatments in preparation for implantation. For example, U.S. Pat. No. 4,755,593 discloses subjecting xenograft tissue to mechanical strain by stretching to produce a thinner and stiffer biomaterial for grafting. Tissue for allograft transplantation is commonly cryopreserved to optimize cell viability during storage, as disclosed, for example, in U.S. Pat. No. 5,071,741; U.S. Pat. No. 5,131,850; U.S. Pat. No. 5,160,313; and U.S, Pat. No. 5,171,660. U.S. Pat. No. 5,071,741 discloses that freezing tissues causes mechanical injuries to cells therein because of extracellular or intracellular ice crystal formation and osmotic dehydration.

Galili et al provide a review article in Xenotransplantation 1997; 4:127-131 entitled "High-affinity anti-Gal immunoglobulin G in chronic rejection of xenografts". Galili et al describe "Porcine and bovine cartilage transplants in cynomolgus monkey" in Transplantation, Vol.. 63, 646-651, No. 5; March 15, 1997.

US Patent No. 5,333,626 discloses the preparation of bone for transplantation.

WO 99/44533 is an international patent application available for the purpose of novelty under Article 54(3) EPC entitled "Soft Tissue Xenografts".

WO 99/47080 is an international patent application available for the purpose of novelty under Article 54(3) EPC entitled "Bone Xenografts".

### SUMMARY OF THE INVENTION

The present invention provides a substantially non-immunogenic bone xenograft for implantation into a human in need of bone repair or replacement. The invention further provides methods for processing xenogeneic bone with reduced immunogenicity but with substantially native elasticity and load-bearing capabilities for xenografting into humans.

As described herein, the term "xenograft" is synonymous with the term "heterograft" and refers to a graft transferred from an animal of one species to one of another species. Stedman's Medical Dictionary, Williams & Wilkins, Baltimore, MD (1995).

As described herein, the term "xenogeneic", as in xenogeneic graft, bone, etc., refers to a graft, bone, etc., transferred from an animal of one species to one of another species. *Id*.

The methods of the invention are defined in claims 1 to 6. The methods of the invention may further include treatment with radiation, one or more cycles of freezing and thawing, treatment with a chemical cross-linking agent, treatment with alcohol or ozonation. In addition to or in lieu of these treatments, the methods of the invention include a cellular disruption treatment. The methods of the invention include the step of digestion of the carbohydrate moieties of the xenograft with a glycosidase in a concentration range of 100 U/ml to 200 U/ml or glycosidase digestion. This may be followed by treatment of the carbohydrate moieties of the xenograft with a sialic acid capping molecule. After one or more of the above-described processing steps, the methods of the invention provide a xenograft having substantially the same mechanical properties as a native bone.

As described herein, the term "cellular disruption" as in, for example, cellular disruption treatment, refers to a treatment for killing cells.

As described herein, the term "capping molecule(s)", refers to molecule(s) which link with carbohydrate chains such that the xenograft is no longer recognized as foreign by the subject's immune system.

In one embodiment, the invention provides an article of manufacture comprising a substantially non-immunogenic bone xenograft for implantation into a human.

In another embodiment, the invention provides a method of preparing a bone xenograft for implantation into a human, which includes removing at least a portion of a bone from a non-human animal to provide a xenograft; washing the xenograft in water and alcohol; and subjecting the xenograft to at least one treatment selected from the group consisting of exposure to ultraviolet radiation, immersion in alcohol, ozonation, and freeze/thaw cycling, whereby the xenograft has substantially the same mechanical properties as a corresponding portion of a native bone.

As described herein, the term "portion", as in, for example, a portion of bone or second surface carbohydrate moieties, refers to all or less than all of the respective bone or second surface carbohydrate moieties.

The invention provides a method of preparing a bone xenograft for implantation into a human, which includes removing at least a portion of a bone from a non-human animal to provide a xenograft; washing the xenograft in water and alcohol; subjecting the xenograft to a cellular disruption treatment; and digesting the xenograft with a glycosidase in a concentration range of about 100 U/ml to about 200 U/ml to remove substantially first surface carbohydrate moieties from the xenograft, whereby the xenograft has substantially the same mechanical properties as a corresponding portion of a native bone wherein the xenograft does not comprisea substantially non-immunogenic soft tissue xenograft.

In a further embodiment, the invention provides a method of preparing a bone xenograft for implantation into a human, which includes removing at least a portion of a bone from a non-human animal to provide a xenograft; washing the xenograft in water and alcohol; subjecting the xenograft to a cellular disruption treatment; digesting the xenograft with a glycosidase to remove substantially first surface carbohydrate moieties from the xenograft; and treating second surface carbohydrate moieties on the xenograft with sialic acid to cap at least a portion of the second surface carbohydrate moieties, whereby the xenograft is substantially non-immunogenic and has substantially the same mechanical properties as a corresponding portion of a native bone.

As described herein, the terms "to cap" or "capping", refer to linking a capping molecule such as a carbohydrate unit to the end of a carbohydrate chain, as in, for example, covalently linking sialic acid to surface carbohydrate moieties on the xenograft.

In still further embodiments, the invention provides articles of manufacture including substantially non-immunogenic bone xenografts for implantation into humans produced by the above-identified methods of the invention.

### BRIEF DESCRIPTION OF THE DRAWINGS

The various features of the invention may be more fully understood from the following description when read together with the accompanying drawings.
FIG. 1 shows a portion of a bone having a defect;
FIG. 2 shows the bone portion of FIG. 1 with a xenograft of the invention in the defect; and
FIG. 3 is a graphical representation of the specificity of monoclonal anti-Gal antibodies for α-galactosyl epitopes on bovine serum albumin (BSA), bovine thyroglobulin, mouse laminin, Galβ1-4 G1cNAc-BSA (*N*-acetyllactosamine-BSA), Galα1-4Galβ1-4G1cNAc-BSA (P1 antigen linked to BSA), and human thyroglobulin or human laminin.

### DETAILED DESCRIPTION OF THE PREFERRED EMBODIMENTS

The present invention is directed against the chronic rejection of xenografts for implantation into humans. Accordingly, the bone xenograft produced in accordance with the method of the invention is substantially non-immunogenic, while generally maintaining the mechanical properties of a native bone.

The bone xenograft may be cut into segments, each of which may be implanted into the recipient as set forth below.

The invention provides, in one embodiment, a method for preparing or processing a xenogeneic bone for engraftment into humans. The bone may be harvested from any non-human animal to prepare the xenografts of the invention. Bone from transgenic non-human animals or from genetically altered non-human animals may also be used as xenografts in accordance with the present invention. Preferably, bovine, ovine, or porcine bones serve as sources of the bone used to prepare the xenografts. More preferably, immature pig, calf or lamb bones are the sources of the bone, since the bone of younger animals has more cancellous bone and may be less brittle than that of older animals. Most preferably, the age of the source animal is between six and eighteen months at time of slaughter.

In the first step of the method of the invention, an intact bone portion is removed from a bone of a non-human animal. The source of the bone should be collected from freshly killed animals and preferably immediately placed in a suitable sterile isotonic or other tissue preserving solution. Harvesting of the bone portions should occur as soon as possible after slaughter of the animal and preferably should be performed in the cold, *i.e.,* in the approximate range of about 5°C to about 20°C, to minimize enzymatic degradation of the bone tissue.

The bone portions are harvested in the cold, under strict sterile technique following known surgical procedures. The harvested bone portion is cut up into strips or blocks and provided with and without cancellous bone attached to cortical bone.

The resultant xenograft is washed in about ten volumes of sterile cold water to remove residual blood proteins and water soluble materials. The xenograft is then immersed in alcohol at room temperature for about five minutes, to sterilize the bone and to remove non-collagenous materials.

After alcohol immersion, the xenograft may be subjected to at least one of the following treatments: radiation treatment, treatment with alcohol, ozonation, one or more cycles of freezing and thawing, and/or treatment with a chemical cross-linking agent. When more than one of these treatments is applied to the xenograft, the treatments may occur in any order.

In one embodiment of the method of the invention, the xenograft may be treated by exposure to ultraviolet radiation for about fifteen minutes or gamma radiation in an amount of about .5 to 3 MegaRad.

In another embodiment, the xenograft may be treated by again being placed in an alcohol solution. Any alcohol solution may be used to perform this treatment. Preferably, the xenograft is placed in a 70% solution of isopropanol at room temperature.

In still another embodiment, the xenograft may be subjected to ozonation.

In a further embodiment of the method of the invention, the xenograft may be treated by freeze/thaw cycling. For example, the xenograft may be frozen using any method of freezing, so long as the xenograft is completely frozen, i.e., no interior warm spots remain which contain unfrozen tissue. Preferably, the xenograft is dipped into liquid nitrogen for about five minutes to perform this step of the method. More preferably, the xenograft is frozen slowly by placing it in a freezer. In the next step of the freeze/thaw cycling treatment, the xenograft is thawed by immersion in an isotonic saline bath at room temperature (about 25 °C) for about ten minutes.

In yet a further embodiment, the xenograft may optionally be exposed to a chemical agent to tan or crosslink the proteins within the extracellular matrix, to further diminish or reduce the immunogenic determinants present in the xenograft. Any tanning or crosslinking agent may be used for this treatment, and more than one crosslinking step may be performed or more than one crosslinking agent may be used in order to ensure complete crosslinking and thus optimally reduce the immunogenicity of the xenograft. For example, aldehydes such as glutaraldehyde, formaldehyde, adipic dialdehyde, and the like, may be used to crosslink the collagen within the extracellular matrix of the xenograft in accordance with the method of the invention. Other suitable crosslinking agents include aliphatic and aromatic diamines, carbodiimides, diisocyanates, and the like.

When glutaraldehyde is used as the crosslinking agent, for example, the xenograft may be placed in a buffered solution containing about 0.05 to about 5.0% glutaraldehyde and having a pH of about 7.4. Any suitable buffer may be used, such as phosphate buffered saline or trishydroxymethylaminomethane, and the like, so long as it is possible to maintain control over the pH of the solution for the duration of the crosslinking reaction, which may be from one to fourteen days, and preferably from three to five days.

Alternatively, the xenograft can be exposed to a crosslinking agent in a vapor form, including, but not limited to, a vaporized aldehyde crosslinking agent, such as, for example, vaporized formaldehyde. The vaporized crosslinking agent can have a concentration and a pH and the xenograft can be exposed to the vaporized crosslinking agent for a period of time suitable to permit the crosslinking reaction to occur. For example, the xenograft can be exposed to vaporized crosslinking agent having a concentration of about .05 to about 5.0% and a pH of about 7.4, for a period of time which can be from one to fourteen days, and preferably from three to five days. Exposure to vaporized crosslinking agent can result in reduced residual chemicals in the xenograft from the crosslinking agent exposure.

The crosslinking reaction should continue until the immunogenic determinants are substantially removed from the xenogeneic tissue, but the reaction should be terminated prior to significant alterations of the mechanical properties of the xenograft. When diamines are also used as crosslinking agents, the glutaraldehyde crosslinking should occur after the diamine crosslinking, so that any unreacted diamines are capped. After the crosslinking reactions have proceeded to completion as described above, the xenograft should be rinsed to remove residual chemicals, and 0.01-0.05 M glycine may be added to cap any unreacted aldehyde groups which remain.

In addition to the above treatments, the xenograft is subjected to a cellular disruption treatment to kill the xenograft's cells, which precedes or follows digestion of the xenograft with glycosidases to remove surface carbohydrate moieties from the xenograft. The glycosidase concentration is in a range 100 U/ml to 200 U/ml. The glycosidase digestion in turn can be followed by linkage with capping molecules such as sialic acid to cap surface N-acetyllactosamine ends of carbohydrate chains of the xenograft.

In an embodiment of this method of the invention, the xenograft is subjected to a cellular disruption treatment to kill the cells of the bone prior to *in vitro* digestion of the xenograft with glycosidases. Typically after surface carbohydrate moieties have been removed from nucleated cells and the extracellular matrix, nucleated, i.e., living cells re-express the surface carbohydrate moieties. Re-expression of antigenic moieties of a xenograft can provoke continued immunogenic rejection of the xenograft. In contrast, non-nucleated, i.e., dead cells, are unable to re-express surface carbohydrate moieties. Removal of antigenic surface carbohydrate moieties from the non-nucleated cells and extracellular matrix of a xenograft substantially permanently eliminates antigenic surface carbohydrate moieties as a source of immunogenic rejection of the xenograft.

Accordingly, in the above-identified embodiment, the xenograft made according to the present invention is subjected to freeze/thaw cycling as discussed above to disrupt, i.e., to kill the cells of the bone. Alternatively, the xenograft of the present invention is treated with gamma radiation having an amount of .2 MegaRad up to about 3 MegaRad. Such radiation kills the bone cells and sterilizes the xenograft. Once killed, the bone cells are no longer able to re-express antigenic surface carbohydrate moieties such α-gal. epitopes which are factors in the immunogenic rejection of the transplanted xenografts.

Either before or after the bone cells are killed, the xenograft is subjected to *in vitro* digestion of the xenograft with glycosidases, and specifically galactosidases, such as α-galactosidase, to enzymatically eliminate antigenic surface carbohydrate moieties. In particular, α-gal epitopes are eliminated by enzymatic treatment with α-galactosidases, as shown in the following reaction: 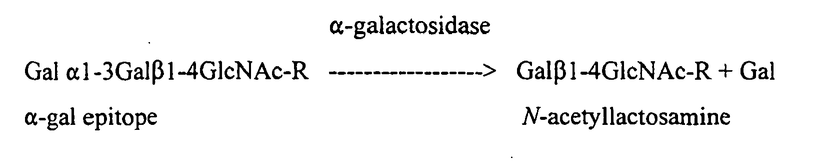 The *N*-acetyllactosamine residues are epitopes that are normally expressed on human and mammalian cells and thus are not immunogenic. The *in vitro* digestion of the xenograft with glycosidases is accomplished by various methods. For example, the xenograft can be soaked or incubated in a buffer solution containing glycosidase. In addition, the xenograft can be pierced to increase permeability, as further described below. Alternatively, a buffer solution containing the glycosidase can be forced under pressure into the xenograft via a pulsatile lavage process.

Elimination of the α-gal epitopes from the xenograft diminishes the immune response against the xenograft. The α-gal epitope is expressed in nonprimate mammals and in New World monkeys (monkeys of South America) as 1x10⁶-35x10⁶ epitopes per cell, as well as on macromolecules such as proteoglycans of the extracellular matrix. U. Galili et al., Man, apes, and Old World monkeys differ from other mammals in the expression of α-galactosyl epitopes on nucleated cells, 263 J. Biol. Chem. 17755 (1988). This epitope is absent in Old World primates (monkeys of Asia and Africa and apes) and humans, however. *Id.* Anti-Gal is produced in humans and primates as a result of an immune response to α-gal epitope carbohydrate structures on gastrointestinal bacteria. U. Galili et al., Interaction between human natural anti-α-galactosyl immunoglobulin G and bacteria of the human flora, 56 Infect. Immun. 1730 (1988); R.M. Hamadeh et al., Human natural anti-Gal IgG regulates alternative complement pathway activation on bacterial surfaces, 89 J. Clin. Invest. 1223 (1992). Since nonprimate mammals produce α-gal epitopes, xenotransplantation of xenografts from these mammals into primates results in rejection because of primate anti-Gal binding to these epitopes on the xenograft. The binding results in the destruction of the xenograft by complement fixation and by antibody dependent cell cytotoxicity. U. Galili et al., Interaction of the natural anti-Gal antibody with α-galactosyl epitopes: A major obstacle for xenotransplantation in humans, 14 Immunology Today 480 (1993); M. Sandrin et al., Anti-pig 1gM antibodies in human serum react predominantly with Galα1-3Gal epitopes, 90 Proc. Natl. Acad. Sci. USA 11391 (1993); H. Good et al., Identification of carbohydrate structures which bind human anti-porcine antibodies: implications for discordant grafting in man. 24 Transplant. Proc. 559 (1992); B.H. Collins et al., Cardiac xenografts between primate species provide evidence for the importance of the α-galactosyl determinant in hyperacute rejection, 154 J. Immunol. 5500 (1995). Furthermore, xenotransplantation results in major activation of the immune system to produce increased amounts of high affinity anti-Gal. Accordingly, the substantial elimination of α-gal epitopes from bone cells and the extracellular matrix, and the prevention of re-expression of cellular α-gal epitopes can diminish the immune response against the xenograft associated with anti-Gal antibody binding with α-gal epitopes.

Following treatment with glycosidase, the remaining carbohydrate chains (e.g., glycosaminoglycans) of the xenograft are optionally treated with capping molecules to cap at least a portion of the remaining carbohydrate chains. This capping treatment involves capping molecules having a concentration range of about 0.1 mM to about 100 mM, and preferably, a concentration of about .1 mM to about 10 mM, and most preferably, a concentration of about 1 mM to about 4 mM. Treatment with capping molecules is applicable to both glycosidase-treated and non-glycosidase-treated xenografts. For example, xenografts from knock out animals which may lack α-gal epitopes may be treated with capping molecules to cap carbohydrate moieties on the xenograft, thereby reducing the xenograft's immunogenicity. Examples of capping molecules used in the present invention include fucosyl and n-acetyl glucosamine and sialic acid.

In addition, selected capping molecules, such as sialic acid, are negatively charged. The replacement of α-gal epitopes with negatively charged molecules can further diminish immunogenic rejection of the xenograft. It is theorized that the decreased immunogenicity of the xenograft results because the negative charges conferred by the capping molecules repel negatively charged antibody molecules and/or cells of the immune system, thereby masking immunogenic regions of the xenograft.

In general, electrostatic repulsion termed "zeta potential," prevents the interaction between molecules, other than ligands and their corresponding receptors, in the body, and serves as a barrier against nonspecific interactions. For example, sialic acid on carbohydrate chains of envelope glycoproteins helps infectious viruses to evade effective recognition by antibodies and by antigen presenting cells. T.W. Rademacher et al., Glycobiology, Ann. Rev. Biochem., 57:785 (1988). Bacteria such as *Neisseria gonorrhea* can prevent their immune destruction by coating themselves with sialic acid using a bacterial sialyltransferase. R.F. Rest et al., Neisseria sialyltransferases and their role in pathogenesis, Microbial Pathogenesis, 19:379 (1995). Similarly, the protozoan *Trypanosoma cruzi* can infect humans and cause Chagas' disease because of effective sialylation of its cell surface glycoproteins with sialic acid by use of the enzyme transialidase which transfers sialic acid from host glycoproteins to carbohydrate chains on the parasite's membrane. O. Previato et al., Incorporation of sialic acid into Trypanosoma cruzi macromolecules, A proposal for new metabolic route, Mol. Biochem. Parasitol., 16:85 (1985); B. Zingales et al., Direct sialic acid transfer from a protein donor to glycolipids of trypomastigote forms of Trypanosoma cruzi, Mol. Biochem. Parasitol., 26:1335 (1987). Decreasing immunogenicity by sialic acid is a method also used by mammalian cells. Normal antigen presenting cells prevent nonspecific adhesion with T lymphocytes by the expression of a highly sialylated protein named sialophorin (also termed CD43). E. Famole-Belasio et al., Antibodies against sialophorin (CD43) enhance the capacity of dendritic cells to cluster and activate T lymphocytes., J. Immunol., 159:2203 (1997). Many malignant cell types that acquire metastatic properties, increase the expression of sialic acid on their cell surface glycoproteins and thus mask their tumor antigens and decrease the possibility of their detection and destruction by the immune system. G. Yogeswarren et al., Metastatic potential is positively correlated with cell surface sialylation of cultural murine cells, Science, 212:1514 (1981); J.W. Dennis, Changes in glycosylation associated with malignant transformation and tumor progression. In: Cell surface carbohydrates and cell development, M. Fukuda, Ed. CRC Press, pp. 161-213 (1992).

The same strategy for prevention of immune recognition can be implemented by treatment of α-galactosidase treated xenografts with negatively charged molecules. The addition of negatively charged molecules to the ends of the carbohydrate chains on the cells and/or on the extracellular matrix molecules of the α-galactosidase treated xenografts can mask the non-α-Gal antigens of the xenograft and diminish immunogenic rejection of the xenograft.

Sialic acid is a non-limiting example of a negatively charged capping molecule used to cap the carbohydrate chains of the xenograft of the present invention. Sialic acid can be linked *in vitro* to carbohydrate chains of the xenograft by sialyltransferase (ST), preferably in a concentration of about 1 mU/ml to about 1000 U/ml, and more preferably in a concentration of about 10 U/ml to about 200 U/ml, in the following exemplary reaction: 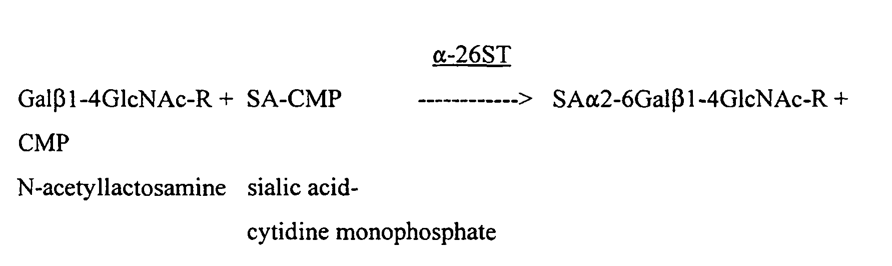 Sialic acid can also be linked *in vitro* to carbohydrate chains of the xenograft by recombinant trans-sialidase (TS), preferably in a concentration of about 1 mU/ml to about 1000 U/ml, and more preferably in a concentration of about 10 U/ml to about 200 U/ml, in the following exemplary reaction:

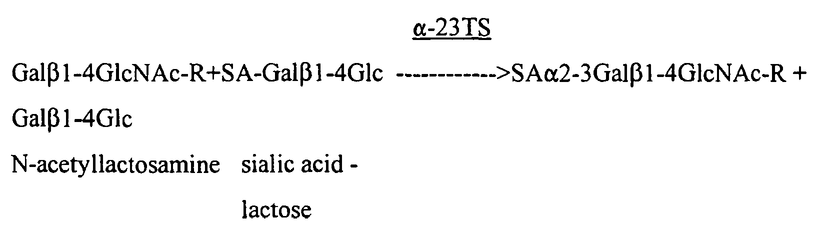

Prior to treatment, the outer lateral surface of the xenograft may optionally be pierced to increase permeability to agents used to render the xenograft substantially non-immunogenic. A sterile surgical needle such as an 18 gauge needle may be used to perform this piercing step, or, alternatively a comb-like apparatus containing a plurality of needles may be used. The piercing may be performed with various patterns, and with various pierce-to-pierce spacings, in order to establish a desired access to the interior of the xenograft. Piercing may also be performed with a laser. In one form of the invention, one or more straight lines of punctures about three millimeters apart are established circumferentially in the outer lateral surface of the xenograft.

Prior to implantation, the bone xenograft of the invention may be treated with limited digestion by proteolytic enzymes such as ficin or trypsin to increase tissue flexibility, or coated with anticalcification agents, antithrombotic coatings, antibiotics, growth factors, or other drugs which may enhance the incorporation of the xenograft into the recipient. The bone xenograft of the invention may be further sterilized using known methods, for example, with additional glutaraldehyde or formaldehyde treatment, ethylene oxide sterilization, propylene oxide sterilization, or the like. The xenograft may be stored frozen until required for use.

Further, the bone xenograft of the invention can be treated with an osteoinductive factor in an effective amount to stimulate the conversion of soft tissue cells to osseous tissue formers. For example, the osteoinductive factor can be added to subcutaneous spaces of the xenograft of the invention at predetermined concentrations. As described herein, the term "osteoinductive factor" refers to a protein which stimulates the differentiation of uncommitted connective tissue cells into bone-forming cells. J.M. Lane et al., Current Approaches to Experimental Bone Grafting, 18 Orthopedic Clinics of North America (2) 214 (1987). Examples of osteoinductive factors which can be used in the present invention include bone morphogenic protein (BMP). Such osteoinductive factors are commercially available.

The bone xenograft of the invention also can be treated with a demineralization agent in an effective amount to remove substantially minerals such as, for example, Calcium from the extracellular matrix of the xenograft. For example, the xenograft of the invention can be soaked in a solution containing demineralization agents, such as , hydrochloric acid, and other demineralization agents known to those of ordinary skill in the art, at predetermined concentrations, to demineralize substantially the xenograft of the invention. Once the minerals are removed from the xenograft, a porous volume matrix is formed with pores ranging in size from about 50 µm (microns) to about 500 µm (microns). It is theorized that the collagen of demineralized extracellular bone matrix serves as an osteoconductive scaffolding and facilitates the migration of bone forming components once bone graft is implanted. J.M. Lane et al., Current Approaches to Experimental Bone Grafting, 18 Orthopedic Clinics of North America (2) 220 (1987). It is further theorized that demineralized bone possesses greater osteoinductive activity than, for example, autologous bone, because bone mineral impedes the release of osteoinductive proteins from extracellular bone matrix. *Id*. at 218. According to this theory, demineralization enlarges the access of surrounding responsive cells to osteoinductive proteins and augments the potential of the osteoinductive proteins.

In addition, a binding agent can be added into the bone xenograft of the present invention. The binding agent is implanted in an effective amount to facilitate the binding of mesenchymal and other bone forming cells to the extracellular matrix of the bone xenograft. For example, the binding agent can be added at predetermined concentrations. Examples of binding agents useful in the present invention include fibronectin protein and RGD peptide, and further include other binding agents known to those persons of ordinary skill in the art.

The bone xenograft of the invention, or a segment thereof, may be implanted into damaged human bones by those of skill in the art using known arthroscopic surgical techniques. Holes in bones are manually packed with bone according to standard surgical techniques Specific instruments for performing surgical techniques are known to those of skill in the art, which ensure accurate and reproducible placement of bone implants.

This invention is further illustrated by the following Examples which should not be construed as limiting.

### EXAMPLE 1: Assay For α-Gal Epitopes' Elimination From Bone By α-Galactosidase

In this example, an ELISA assay for assessing the elimination of α-gal epitopes from bone is conducted.

A monoclonal anti-Gal antibody (designated M86) which is highly specific for α-gal epitopes on glycoproteins is produced by fusion of splenocytes from anti-Gal producing knock-out mice for α 1,3 galactosyltransferase, and a mouse hybridoma fusion partner.

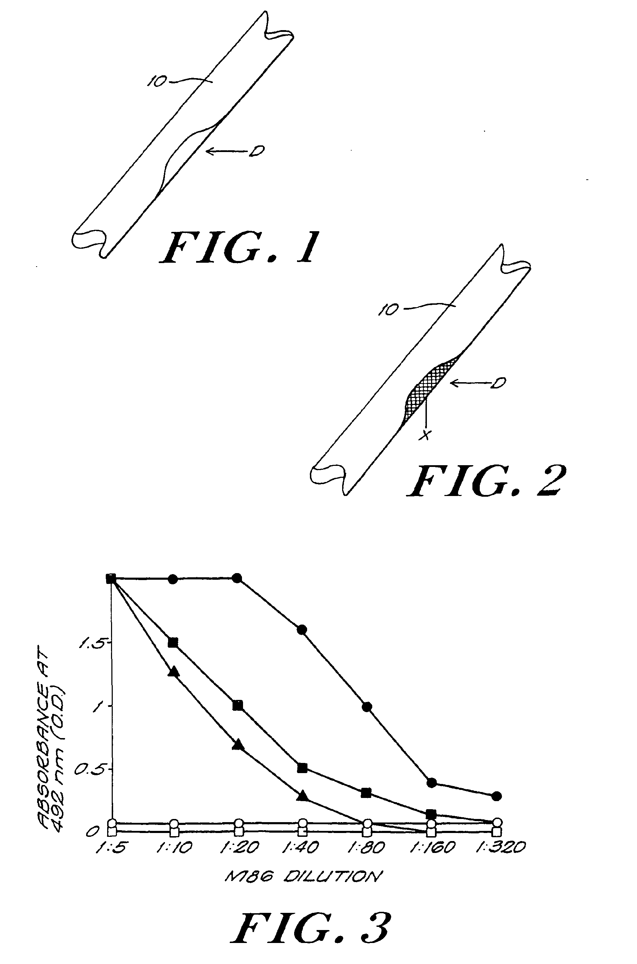

The specificity of M86 for α-gal epitopes on glycoproteins is illustrated in FIG. 3. M86 binds to synthetic α-gal epitopes linked to ●-bovine serum albumin (BSA), to ▲-bovine thyroglobulin which has 11 α-gal epitopes, R.G. Spiro et al., Occurrence of α-D-galactosyl residues in the thyroglobulin from several species. Localization in the saccharide chains of complex carbohydrates, 259 J. Biol. Chem. 9858 (1984); or to ■-mouse laminin which has 50 α-gal epitopes, R.G. Arumugham et al., Structure of the asparagine-linked sugar chains of laminin. 883 Biochem. Biophys. Acta 112 (1986); but not to □-human thyroglobulin or human laminin, ○-Galβ1-4 G1cNAc-BSA (*N*-acetyllactosamine-BSA) and Galα1-4Galβ1-4G1cNAc-BSA (P1 antigen linked to BSA), all of which completely lack α-gal epitopes. Binding is measured at different dilutions of the M86 tissue culture medium.

Once the M86 antibody is isolated, the monoclonal antibody is diluted from about 1:20 to about 1:160, and preferably diluted from about 1:50 to about 1:130. The antibody is incubated for a predetermined period of time ranging between about 5 hr to about 24 hr, at a predetermined temperature ranging from about 3 °C to about 8°C. The antibody is maintained in constant rotation with fragments of bone of about 5 µm to about 100 µm in size, and more preferably with bone fragments ranging from about 10 µm to about 50 µm in size, at various bone concentrations ranging from about 200 mg/ml to about 1.5 mg/ml. Subsequently, the bone fragments are removed by centrifugation at centrifugation rate ranging from about 20,000 x g to about 50,000 x g. The proportion of M86 bound to the bone is assessed by measuring the remaining M86 activity in the supernatant, in ELISA with α-gal-BSA as described in the prior art in, for example, U. Galili et al., Porcine and bovine cartilage transplants in cynomolgus monkey: II. Changes in anti-Gal response during chronic rejection, 63 Transplantation 645-651 (1997). The extent of binding of M86 to the bone is defined as a percentage inhibition of subsequent binding to α-gal-BSA. There is a direct relationship between the amount of α-gal epitopes in the bone and the proportion of M86 complexed with the bone fragments, thus removed from the supernatant (*i.e.,* percentage inhibition).

### EXAMPLE 2: Assessment Of Primate Response To Implanted Bone Treated With α-Galactosidase

In this example, porcine bone implants are treated with α-galactosidase to eliminate α-galactosyl epitopes, the implants are transplanted into cynomolgus monkeys, and the primate response to the implants is assessed. An exemplary bone portion 10 with a defect D is shown in FIG. 1.

Porcine bone specimens are sterilely prepared and the surrounding attached soft tissues surgically removed. The bone specimens are washed for at least five minutes with an alcohol, such as ethanol or isopropanol, to remove synovial fluid and lipid soluble contaminants.

The bone specimens are frozen at a temperature ranging from about -35 °C to about - 90 ° C, and preferably at a temperature up to about -70 ° C, to disrupt, that, is to kill, the specimens' bone cells.

Each bone specimen is cut into two portions. The first bone portion is immersed in a buffer, such as citrate buffer solution, with a pH ranging from about 5 to about 6. The buffer contains α-galactosidase at a concentration ranging from about 50 U/ml to about 300 U/ml and an additive, such as polyethylene glycol (PEG), ranging in a concentration of about 2% to about 6%. The bone/α-galactosidase buffer solution is incubated at a temperature ranging from about 25 °C to about 32 °C for a predetermined period of time ranging from about one hr to about six hr.

The second bone portion is incubated under similar conditions as the first bone portion in a buffer solution in the absence of α-galactosidase and serves as the control.

At the end of incubation, the bone portions are washed under conditions which allow the enzyme to diffuse out. For example, in the present example, the bone portions are washed twice with citrate buffer and three times with phosphate-buffered saline (PBS) pH 7.5. Each wash can include incubation in 50 ml of buffer solution for 10 min with gentle rocking at 24°C. Other washing procedures known to those of ordinary skill in the art can also be used.

Confirmation of complete removal of α-gal epitopes is performed using the ELISA inhibition assay with the monoclonal anti-Gal M86 antibody, as described above in Example 1. The α-galactosidase is produced according to the methods known in the prior art, such as, for example, the methods described in A. Zhu et al., Characterization of recombinant α-galactosidase for use in seroconversion from blood group B to O of human erythrocytes, 827 Arch. Biochem. Biophysics 324 (1996); A. Zhu et al., High-level expression and purification of coffee bean α-galactosidase produced in the yeast Pichia pastoris, 827 Arch. Biochem. Biophysics 324 (1996).

The bone samples are implanted in six cynomolgus monkeys under general inhalation anesthesia following known surgical procedures. Bone is implanted in subcutaneous tissues to evaluate the osteoinductive properties of bone. Any bone formed is evidence of osteoinductive properties. Osteoconductive properties of bone xenograft are evaluated after the xenograft is implanted using bone defective models such as calveria model (skull hole model) and long bone drill hole model. The implantation procedure is performed under sterile surgical technique, and the wounds are closed with 3-0 vicryl or a suitable equivalent known to those of ordinary skill in the art. FIG. 2 shows the bone portion 10 with the xenograft X (shown crosshatched) in place at the defect D. The animals are permitted unrestricted cage activity and monitored for any sign of discomfort, swelling, infection, or rejection. Blood samples (e.g., 2 ml) are drawn periodically (e.g., every two weeks) for monitoring of antibodies.

The occurrence of an immune response against the xenograft is assessed by determining anti-Gal and non-anti-Gal anti-bone xenograft antibodies (*i.e.,* antibodies binding to antigens other than the α-gal epitopes) in serum samples from the transplanted monkeys. At least two ml blood samples are drawn from the transplanted monkeys on the day of implant surgery and at periodic (e.g.. two week) intervals post-transplantation. The blood samples are centrifuged and the serum samples are frozen and evaluated for the anti-Gal and other non-anti-Gal anti-bone xenograft antibody activity.

Anti-Gal activity is determined in the serum samples in ELISA with α-gal-BSA as solid phase antigen, according to methods known in the prior art, such as, for example, the methods described in Galili et al., Porcine and bovine cartilage transplants in cynomolgus monkey: II. Changes in anti-Gal response during chronic rejection, 63 Transplantation 645-651 (1997). For example, the α-gal-BSA antigen is used to coat ELISA microtiter wells. Subsequent to blocking of the wells with 1% BSA in PBS, sera is added to the wells in two fold serial dilutions, and incubated for 2 hr at room temperature. The plates are washed, and incubated with secondary anti-IgG antibody conjugated to peroxidase. Color reaction is performed with o-phenylenediamine. Anti-Gal activity at the various post-transplantation serum dilutions are compared with the baseline pretransplantation serum.

Assays are conducted to determine whether α-galactosidase treated xenografts induce the formation of anti-bone xenograft antibodies. For measuring anti-bone xenograft antibody activity, an ELISA assay is performed according to methods known in the prior art, such as, for example, the methods described in K.R. Stone et al., Porcine and bovine cartilage transplants in cynomolgus monkey: I. A model for chronic xenograft rejection, 63 Transplantation 640-645 (1997). For example, a solution of bone homogenate at 100 µg/ml in carbonate buffer is used as solid phase antigen. Other buffers known to those of ordinary skill in the art can also be used. Approximately 5 µg of bone antigens per well are dried and the wells are blocked with BSA. The serum samples used for this assay are depleted of anti-Gal by adsorption on rabbit red cells for 30 min at 4°C (at 3:1 ration vol/vol). Under these conditions, all anti-Gal antibodies are adsorbed on the many α-gal epitopes expressed on rabbit red cells. U. Galili et al., Evolutionary relationship between the anti-Gal antibody and the Galα163Gal epitope in primates, 84 Proc. Natl. Acad. Sci. (USA) 1369 (1987); U. Galili et al., Contribution of anti-Gal to primate and human IgG binding to porcine endothelial cells, 60 Transplantation 210 (1995). The adsorbed sera at various dilutions are analyzed for anti-cartilage antibodies by ELISA, and the post-transplantation production of such antibodies are assessed by comparing this antibody activity with that observed in the pretransplantation serum.

The bone xenografts are optionally explanted at one to two months post-transplantation, sectioned and stained for histological evaluation of inflammatory infiltrates. Post-transplantation changes in anti-Gal and other anti-bone xenograft antibody activities are correlated with the inflammatory histologic characteristics (i.e., granulocytes or mononuclear cell infiltrates) within the explanted bone, one to two months post-transplantation, using methods known in the art, as, for example, the methods described in K.R. Stone et al., Porcine and bovine cartilage transplants in cynomolgus monkey: I. A model for chronic xenograft rejection, 63 Transplantation 640-645 (1997).

Where the bone xenograft is explanted, the bone xenograft is aseptically harvested, using anesthetic procedure, surgical exposure of the bone, removal of the implant and closure of the soft tissue. The xenograft samples are collected, processed, and examined microscopically. A portion of the implant and surrounding tissue is frozen in an embedding medium for frozen tissue specimens in embedding molds for immunohistochemistry evaluation according to the methods known in the prior art. "TISSUE-TEK®" O.C.T. compound which includes 10.24% w/w polyvinyl alcohol, 4.26% w/w polyethylene glycol, and 86.60% w/w nonreactive ingredients, and is manufactured by Sakura FinTek, Torrence, California, is a non-limiting example of a possible embedding medium for use with the present invention. Other embedding mediums known to those of ordinary skill in the art may also be used. The remaining implant and surrounding tissue is collected in 10% neutral buffered formalin for histopathologic examination.

### EXAMPLE 3: Assessment Of Primate Response To Implanted Bone Treated With α-Galactosidase, Sialic Acid-Cytosine Monophosphate and Sialyltransferase

In this example, porcine bone implants are treated with α-galactosidase to eliminate α-gal epitopes, as described in Example 2. The implants are further treated with sialic acid- cytosine monophosphate (SA-CMP) and sialyltransferase to cap carbohydrate chains with sialic acid. Sialytransferase facilitates the transfer of the sialic acid from the SA-CMP compound to the xenograft. The sialic acid links to and thus caps the carbohydrate chains. The cytosine monophosphate provides the necessary energetic level to the sialic acid for such linking and capping. Capping with sialic acid interferes with the ability of the subject's immune system to recognize the xenograft as foreign. The negative charge of the sialic acid further interferes with the ability of the bone antigens to bind with non-anti-Gal anti-bone antibodies (*i.e.,* antibodies binding to bone antigens other than the α-gal epitopes.) The implants are transplanted into cynomolgus monkeys, and the primate response to the bone implants is assessed.

Porcine bone specimens are prepared as described in Example 2 including the α-galactosidase treatment. Prior to implantation into the monkeys, however, the implants are further treated with a predetermined amount of SA-CMP and sialyltransferase, at specified concentrations for a predetermined time and at a predetermined temperature, to cap carbohydrate chains with sialic acid. For example, the sample is immersed in 10 ml buffer solution at a pH of about 5.5 to 7.0, and preferably a pH of about 6.0-6.5, and most preferably a pH of about 6.2, containing SA-CMP at a concentration of approximately about 1 mM to about 10 mM, and sialyltransferase at a concentration of about 100 U/ml. The sample is incubated at a temperature range of about 26°C to about 37°C for a predetermined time period of about one hr to about four hr.

Other enzymes such as recombinant transialidase can be used to facilitate the transfer of sialic acid from compounds such as sialylated lactose to the xenograft.

Further, other molecules, such as fucosyl in combination with the corresponding fucosyltransferase and n-acetyl glucosamine in combination with the corresponding glycosyltransferase, can also be used for capping the carbohydrate chains of the implants.

Subsequently, the samples are washed to remove the enzyme and implanted into the monkeys, and the occurrence of an immune response against the xenograft is assessed as described above in Example 2.

## Claims

1. A method of preparing a bone xenograft for implantation into a human, which comprises:
a. removing at least a portion of a bone from a non-human animal to provide a xenograft;
b. washing the xenograft in water and alcohol;
c. subjecting the xenograft to a cellular disruption treatment; and
d. digesting the xenograft with a glycosidase to remove substantially a plurality of first surface carbohydrate moieties from the xenograft, wherein the glycosidase has a concentration in a range of 100 U/ml to 200 U/ml, and whereby the xenograft has substantially the same mechanical properties as a corresponding portion of a native bone, wherein the xenograft does not comprise a substantially non-immunogenic soft-tissue xenograft.

2. The method of claim 1, further comprising the step of:
subsequent to the glycosidase digesting step, treating a plurality of second surface carbohydrate moieties on the xenograft with a plurality of capping molecules to cap at least a portion of the second surface carbohydrate moieties, whereby the xenograft is substantially non-immunogenic.

3. The method of claim 2, wherein the capping step comprises treating the second surface carbohydrate moieties on the xenograft with the capping molecules having a concentration in a range of 0.1 mM to 100 mM, and/or at least a portion of the capping molecules are sialic acid molecules.

4. The method of claim 1, wherein the glycosidase is a galactosidase; optionally wherein the galactosidase is an α-galactosidase.

5. The method of claim 1, wherein the cellular disruption treatment comprises;
(i) freeze/thaw cycling; or
(ii) exposure to gamma radiation.

6. The method of claim 1 further comprising the step of following step c:
(i) exposing the xenograft to a crosslinking agent in a vapor form; or
(ii) treating the xenograft with a demineralization agent to remove substantially minerals from an extracellular matrix; or
(iii) adding an osteoinductive factor to the xenograft; or
(iv) adding a binding agent to the xenograft.

7. An article of manufacture comprising a substantially non-immunogenic knee bone xenograft for implantation in to a human obtainable by the method of any one of claims 1 to 6.

## Patentansprüche

1. Verfahren zur Herstellung eines Knochen-Xenotransplantats zur Implantation in einen Menschen, welches umfasst:
a) Entnehmen von mindestens einem Anteil eines Knochens aus einem nicht menschlichen Tier, um ein Xenotransplantat bereitzustellen;
b) Waschen des Xenotransplantats in Wasser und Alkohol;
c) Unterwerfen des Xenotransplantats einer Zellaufbruchbehandlung; und
d) Digestion des Xenotransplantats mit einer Glycosidase, um im Wesentlichen eine Mehrzahl von ersten Oberflächen-Kohlehydrateinheiten von dem Xenotransplantat zu entfernen, wobei die Glycosidase eine Konzentration in einem Bereich von 100 U/ml bis 200 U/ml aufweist und wobei das Xenotransplantat im Wesentlichen die gleichen mechanischen Eigenschaften wie ein entsprechender Teil eines nativen Knochens aufweist, wobei das Xenotransplantat nicht ein im Wesentlichen nicht-immunogenes Weichgewebe-Xenotransplantat umfasst.

2. Verfahren nach Anspruch 1, welches weiterhin den Schritt umfasst:
im Anschluss an den Glucosidase-Digestionsschritt Behandeln einer Mehrzahl von zweiten Oberflächen-Kohlehydrateinheiten auf dem Xenotransplantat mit einer Mehrzahl von Capping-Molekülen, um mindestens einen Teil der zweiten Oberflächen-Kohlehydrateinheiten zu kappen, wobei das Xenotransplantat im Wesentlichen nicht-immunogen ist.

3. Verfahren nach Anspruch 2, wobei der Kappungsschritt die Behandlung der zweiten Oberflächen-Kohlehydrateinheiten auf dem Xenotransplantat mit den Capping-Molekülen mit einer Konzentration in einem Bereich von 0,1 mM bis 100 mM umfasst, und/oder mindestens ein Teil der Capping-Moleküle Sialinsäuremoleküle sind.

4. Verfahren nach Anspruch 1, worin die Glycosidase eine Galactosidase ist; wahlweise die Galactosidase eine α-Galactosidase ist.

5. Verfahren nach Anspruch 1, worin die Zellaufbruchbehandlung umfasst:
(i) Gefrier/Tau-Cycling; oder
(ii) Aussetzen gegenüber Gammastrahlung.

6. Verfahren nach Anspruch 1, welches weiterhin den Schritt des folgenden Schritts c umfasst:
(i) Aussetzen des Xenotransplantats gegenüber einem Vernetzungsmittel in dampfförmiger Form; oder
(ii) Behandlung des Xenotransplantats mit einem Entmineralisierungsmittel, um im Wesentlichen Mineralien aus einer extrazellulären Matrix zu entfernen; oder
(iii) Zugabe eines osteoinduktiven Faktors zu dem Xenotransplantat; oder
(iv) Zugabe eines Bindemittels zu dem Xenotransplantat.

7. Fertigungsgegenstand, der ein im Wesentlichen nicht immunogenes Knieknochen-Xenotransplantat zur Implantation in einen Menschen umfasst, das durch das Verfahren nach einem der Ansprüche 1-6 erhältlich ist.

## Revendications

1. Procédé de préparation d'une xénogreffe osseuse pour l'implantation chez un sujet humain, qui comprend les étapes consistant à :
a) prélever au moins une partie d'un os chez un animal non humain afin d'obtenir une xénogreffe ;
b) laver la xénogreffe dans de l'eau et de l'alcool ;
c) soumettre la xénogreffe à un traitement de dissociation cellulaire ; et
d) digérer la xénogreffe avec une glycosidase afin d'éliminer pratiquement une pluralité de premiers fragments hydrates de carbone superficiels de la xénogreffe, la glycosidase ayant une concentration dans une plage de 100 U/ml à 200 U/ml, grâce à quoi la xénogreffe possède pratiquement les mêmes propriétés mécaniques qu'une partie correspondante d'un os natif, la xénogreffe ne comprenant pas de xénogreffe de tissu mou pratiquement non immunogène.

2. Procédé selon la revendication 1, comprenant en plus l'étape consistant à :
après l'étape de digestion par la glycosidase, traiter une pluralité de deuxièmes fragments hydrates de carbone superficiels sur la xénogreffe avec une pluralité de molécules de coiffage pour coiffer au moins une partie des deuxièmes fragments hydrates de carbone superficiels, grâce à quoi la xénogreffe est pratiquement non immunogène.

3. Procédé selon la revendication 2, dans lequel l'étape de coiffage comprend le traitement des deuxièmes fragments hydrates de carbone superficiels sur la xénogreffe avec les molécules de coiffage ayant une concentration dans une plage de 0,1 mM à 100 mM et/ou dans lequel au moins une partie des molécules de coiffage sont des molécules d'acide sialique.

4. Procédé selon la revendication 1, dans lequel la glycosidase est une galactosidase, la galactosidase étant le cas échéant une α-galactosidase.

5. Procédé selon la revendication 1, dans lequel le traitement de dissociation cellulaire comprend :
(i) des cycles de congélation/décongélation ; ou
(ii) l'exposition à un rayonnement gamma.

6. Procédé selon la revendication 1 comprenant en plus, la suite de l'étape c), l'étape consistant à :
(i) exposer la xénogreffe à un agent de réticulation sous forme de vapeur ; ou
(ii) traiter la xénogreffe avec un agent déminéralisant afin d'éliminer pratiquement les minéraux d'une matrice extracellulaire ; ou
(iii) ajouter un facteur ostéo-inducteur à la xénogreffe ; ou
(iv) ajouter un agent de liaison à la xénogreffe.

7. Article manufacturé comprenant une xénogreffe osseuse de genou pratiquement non immunogène pour l'implantation chez un sujet humain, pouvant être obtenu par le procédé selon l'une quelconque des revendications 1 à 6.
